# EUROPEAN PATENT SPECIFICATION

(11) **EP 1 359 837 B1**
(45) Date of publication and mention of the grant of the patent: **22.05.2013**
(21) Application number: 01271174.3
(22) Date of filing: 19.12.2001
(51) Int. Cl.: A61B 5/00, A61B 5/15, A61B 10/00

(54) **ANALYTE MEASUREMENT**
ANALYTMESSUNG
MESURE D'ANALYTE

(30) Priority: 19.12.2000 GB 0030929
(43) Date of publication of application: 12.11.2003
(60) Divisional application: 06076969.2
(73) Proprietor: Lifescan Scotland Ltd, Inverness, IV2 3ED, Scotland (GB)
(72) Inventor: STIENE, Matthias, Inverness IV2 3QG (GB); RICHTER, Tanja, Inverness IV2 4SN (GB); ALLEN, John, Mendota Heights, MN 55118 (US); MCALEER, Jerome, Grove, Oxfordshire (GB)
(74) Representative: Mercer, Christopher Paul
(86) International application number: PCT/GB2001/005634
(87) International publication number: WO 2002/049507

(56) References cited:
- WO-A-00/22977
- WO-A-01/64105
- WO-A-95/10221
- WO-A-97/19344
- WO-A1-00/74766
- WO-A1-01/36666
- WO-A1-95/33504
- US-A- 4 873 993
- US-A- 5 801 057
- US-A- 6 083 196
- US-B1- 6 271 040

## Description

### I. Background of the Invention

This invention relates to apparatus for measuring glucose levels in body fluids such as cutaneous (interstitial) fluid.

Over recent years there have been two obvious trends in diagnostic device development: simplification of the test procedure and reduction in the volume of sample required to perform the test. Test simplification allows the assay to be performed by relatively untrained personnel in a "non-laboratory" setting. Thus, for example, cardiac marker tests configured in a lateral flow immunoassay format with labelled antibody allow for early assessment of potential myocardial infarct.

The driving force for the development of tests requiring smaller sample volumes is the reduction of discomfort of the patient. This is particularly important in home tests where, for example, if a glucose test is less painful the user will test more frequently. It is now well documented that diabetics who monitor their blood sugar frequently achieve better glycaemic control and so avoid long-term complications of the disease. With this thought in mind, a number of companies have developed test devices requiring progressively smaller sample volumes, thereby minimising pain.

Some proposals have been made to attempt to reduce the amount of pain involved by with so-called minimally invasive devices (e.g. Integ US patent numbers 5,746,217; 5,820,570; and 5,582,184). Unfortunately, such devices appear to be unable to provide sufficient fluid sample to provide a reliable result and thus, as yet, none has been commercially realised.

In the context of glucose measurement, so-called continuous monitors are also known and these have certain advantages over the 'snapshot' devices outlined above since they provide a clearer insight into trending, the effect of food or medication and overall glycaemic control. Such known devices however suffer from a number of drawbacks, mainly associated with the need to recalibrate the device regularly. This entails performing regular manual tests using a conventional test strip. This can negate many of the advantages of using a continuous device since the user is still relied upon to take action in order for the device to operate properly. Furthermore the accuracy of the devices tends to drift unpredictably between calibrations. The regular finger pricks are also painful. Examples of continuous monitoring sensors include International patent application PCT/DE99/03126 (International Publication WO 00/22977 published 27 April 2000) and International patent application PCT/US99/16378 (International Publication WO 00/04832 published 3 February 2000).

WO00/22977A discloses a minimally invasive sensor system for determining the concentration of substances in the human body.

WO01/64105A discloses an apparatus for detection and quantitation of an electrochemically-detectable analyte, such as glucose, in blood or interstitial fluid.

US5801057 discloses a minimally intrusive and less-painful, self-use microsampling device and method for the measurement of glucose and other analytes in blood.

WO95/33504A discloses an IC processed microneedle including an interface region, and a shaft.

The document discloses a device according to the preamble of claim 1.

WO00/74766A discloses a hollow microneedle with a substantially sharp edge.

US6083196 discloses a device comprising a sheet member having a plurality of microprotrusions for penetrating the skin and a substantially incompressible agent reservoir housing contacting and extending across the sheet member.

### II. Summary and Objects of the disclosure.

It is an object of the invention to provide an improved arrangement and when viewed from a first aspect the invention provides a device for measuring the concentration of an analyte in a fluid; as defined in claim 1. Further aspects are defined in the dependent claims.

A microchannel is used to convey the sample fluid to the sensing means.

As used herein the term "microchannel" refers to a channel, of any suitable cross-section, whose lateral dimension is between approximately 10-500 µm.

Such microchannels are beneficial for a number of reasons in the context of an analyte sensing device. In the context of the measurement of a bodily fluid a small sample volume is beneficial since it means that it is easier to provide a sufficient volume for a valid test. Most importantly the volume of fluid required to carry out an assay is correspondingly small, in addition the microchannel allows to handle flow rates between 10 and 500 nL/min with a high resolution of the measurement over time, due to the low channel volume. The glucose sensing means can be arranged in any suitable configuration depending on the type of sensing means used (electrochemical, optochemical etc.). Preferably the analyte sensing means comprises one or more reagents to react with said analyte and thereby give a measurable output. Such a reagent may be located anywhere, but preferably the reagent or at least one of the reagents is provided on a wall of the microchannel. This arrangement is beneficial since it means that no additional volume is required in order for the sample liquid to be brought fully into contact with the reagent - i.e. the contact area is optimised. When viewed from a second aspect the invention provides a device for measuring the concentration of an analyte in a fluid, comprising a support member and a microchannel provided on said support member for conveying said fluid across the support member, wherein one or more reagents for reacting with said analyte is coated on a wall of the microchannel.

Such a device comprises means for sensing the analyte - for example an electrochemical or a photometric detector.

The devices set out hereinabove may be used for measuring glucose in interstitial fluid. Of course, a suitable sample of bodily fluid may be applied to the device. Preferably however the device is integrated with means for extracting the fluid. Most preferably this comprises means for penetrating the skin - in the case where the sample fluid is interstitial fluid. Most preferably it comprises a skin penetration member such as hollow needle, solid lance or the like.

In accordance with the embodiments set out above the device can be used both to collect the sample fluid and to measure the concentration of the target analyte - or at least give an output from which the concentration can be measured. The present invention provides a device for measuring the concentration of an analyte in a fluid, as defined in claim 1.

Thus it will be seen that in accordance with this disclosure a fluid extraction means, preferably a skin penetration means, most preferably a needle, lance or the like, is provided on a support member of a device for measuring analyte concentration. This allows, in at least preferred cases, the combination of skin penetration means, e.g. needle, and support member, e.g. test strip, to be made disposable and thus as hygienic as possible. The preferred cases require a user merely to puncture their skin with a needle or the like and then blood or interstitial fluid may be conducted automatically onto or into the test member. Devices for extracting interstitial fluid are shown in the afore-mentioned Integ patents and International patent application PCT/US01/09673 (International Publication WO 01/72220 published 4 October 2001).

In such cases the combined needle/test member would normally be used in conjunction with a separate, non-disposable, measuring device for measuring the analyte concentration e.g. by means of the output of an analyte-reagent reaction, where appropriate.

The penetration member is of such a length that it penetrates substantially the cutaneous layer of skin. When the term "substantially the cutaneous layer" is referred to, it means that whilst it is the intention to sample fluid from the cutaneous layer, it cannot be ruled out that some sampling from the sub-cutaneous may occur. The cutaneous layer has a high density of blood capillaries which helps to ensure that the levels of analytes in interstitial fluid reliably reflect those in the blood.

While not limited to analysing interstitial fluid (ISF), the present invention is preferably used in obtaining and analysing ISF. A further benefit offered by interstitial fluid is that it is not as complex as whole blood and therefore does not, for example, suffer from Haematocrit fluctuations. ISF also has a low viscosity and is more suited to passage through microchannels than whole blood. The low sample volume requirement of a microchannel also enables the device reliably to use interstitial fluid.

Perhaps more importantly such shallow skin penetration (cutaneous or subcutaneous) substantially reduces the pain experienced since there is a much lower density of nerve endings than at the depths penetrated by standard needles when extracting blood. Indeed, in at least preferred embodiments, little or no pain is normally felt. This makes prolonged and/or repeated penetration of the skin much more acceptable.

The actual length of the penetration means will depend on the angle at which it is intended to be inserted. Preferably however substantially perpendicular insertion is intended and the length is less than 2 mm, preferably less than 1.5 mm. In some preferred embodiments a length of approximately 1.4 mm is appropriate. However for certain applications a length as short as 0.5mm may be preferred.

Of course a given test member may have just one skin penetration member, or alternatively a plurality may be provided.

The penetration means may comprise a sufficiently short standard needle, possibly shortened if necessary. More preferably the penetration means comprises a microneedle. A microneedle is hereby defined as a needle with a length sufficient to penetrate the cutaneous layer of human skin without substantially penetrating the subcutaneous layer. Such a needle typically has length less than 2mm, preferably between 0.4 and 1.6mm.

The outer diameter is preferably less than 0.5mm, most preferably between 0.1 and 0.3 mm.

The penetration means is integrated with the support member. The comments above regarding this arrangement apply, with the additional benefit noted here that preferred embodiments of this arrangement can provide a compact disposable device which gives rise to substantially no pain, in use and obviates the need for a user to come into contact with or even see the bodily fluid concerned.

Preferred embodiments of the invention use a reagent-based measurement. Many different reagent-based analyte measurements are available to those skilled in the art, allowing the principles of the invention to find wide application. For example an optoohemical- i. e. either fluorescent or luminescent - or an electrochemichal technique could be used.

One preferred embodiment comprises analyte sensing means comprising a mediated amperometric enzyme electrode. For example, a ferrocene-mediated electron transfer from a glucose-oxidase catalysed reaction is a suitable means for detecting the level of glucose in a body fluid. It will be appreciated that use of the above electron-transfer mediator is a non-limiting example and that other electron transfer mediators well known in the art could be used, for example, hexa-cyanoferrate (ferricyanide), oxygen or components of the respiratory chain (i.e. cytochromes)

Preferably the embodiment will comprise an analyte sensing means which operates in conjunction with a test meter to give the measurement. Alternatively the member may comprise the appropriate reagent or dye for performing a colorimetric test in association with light sensitive means in the test meter. It will be seen that such arrangements are consistent with the test device being disposable since the costly elements of the sensing mechanism, such as the light sensitive means, electronic circuity etc., can be placed in a non-disposable test meter.

Just single analyte sensing means may be provided, but preferably more than one is provided.

Thus it will be seen that in accordance with this aspect of the disclosure, a plurality of conduits direct fluid to be measured to respective analyte sensing means. This means that a plurality of measurements of fluid may be made by a single such device. The sensing means may be different so as to measure or test for different analytes in the fluid. Preferably however they are the same or at least are for measuring the same analyte. When viewed from a further aspect the disclosure provides a device for measuring the concentration of an analyte in a fluid, comprising a support member and a plurality of analyte sensing means provided thereon for measuring said concentration, wherein said device further comprises a plurality of conduits such that each of said sensing means has a conduit associated therewith, said conduits serving in use to direct said fluid to respective sensing means. The device, whether it comprises a single or a plurality of conduit/analyte sensors, may be used to continuously measure the concentration of the analyte over a period of time. Periodic measurements may be taken i.e. the fluid is "sample" at various periods over time, for example every 30 minutes. Fluid may continuously flow through the device or may be temporarily stopped by, for example, hydrophobic gates. As yet another alternative, the sensors may be of the single measurement type, after which the device is discarded or fluid is diverted to another conduit to perform another measurement. The total time taken to complete a measurement cycle or the time taken between measurements could vary and would depend upon the degree of monitoring or the analyte of interest. The plurality of conduits would allow for switching from sensor to another after a particular period of time, by diverting fluid flow from one conduit to another. Switching enables measurements to be performed without interruption to the fluid extraction or without having to recalibrate the sensors, which are known to drift after a period of time due to factors such as electrode fouling etc.

Thus analyte monitoring may be made on a truly continuous basis, without the user having to recalibrate or interact with the system. An advantage of performing a single measurement, either in the case of a device with a single or plurality of microchannels is that drifting of the signal over time is no longer an issue that has to be considered and consequently simpler reagent chemistries may be employed in the analyte sensors. For example in the case of an electrochemical detection system, results may be obtained in as little as five seconds or less. Furthermore, such devices would not require storage reservoirs for collection of the waste fluid, since the waste fluid would be stored in the microchannel conduits themselves.

In principle, there would be no upper limit to the number of sensor/conduits contained within a device, the upper limit being determined by factors such as the desired total length of time for analyte measurement or by the size of the device.

Whilst the above disclosure describes a flow-through sensor, i.e. the analyte is measured whilst flowing past the detection means, measurements may also be conducted whilst the fluid is stationary. This could be achieved by the use of flow controlling means such as a hydrophobic gate or gates being activated to temporarily stop the flow of fluid through the microchannel once it had passed the sensing means. Isolating or shutting off a volume of fluid from the rest of the fluid sample would enable an end-point determination of the total amount of analyte present in the particular portion of the sample to be made.

As described above, the sensing means may be electrochemical or non-electrochemical in nature - e.g. of the fluorescent or chemi-luminescent colorimetric sort. For example the sensing means may comprises an enzyme-coated electrode in the case of electrochemical measurement, or in the case of fluorescent colorimetric sensing the sensing means would comprise a suitable reagent dye. It will, of course, be understood that the term 'sensing means' does not necessarily refer to a complete assembly for giving a final reading, but rather to a means on the support member which yields an output which may be read to give a measure of the analyte concentration, e.g. by a separate test meter.

Such devices according the to disclosure as set out above could be arranged to be used in a mode similar to conventional test strips i.e. where a single fluid sample is placed on the device and is measured.

In common with earlier aspects of the disclosure, devices of the sort set out above may be arranged to measure the concentration of any suitable analyte. In all such cases, the analyte's concentration may simply be an indirect indication of the property of the sample fluid which it is desired to monitor.

Preferably however, the device is arranged to measure an analyte concentration directly - i.e. it is the concentration itself which is being monitored. An example of such a measurement would be glucose in blood, the concentration of which is an important parameter for those suffering from diabetes.

The measuring device is suitable for measuring the concentration of glucose, in interstitial fluid. In such an embodiment the device is preferably suitable for attachment to the skin of the subject who is to be measured. The subject may be an animal but is preferably human.

By providing a device which can be attached to a subject, measurements of the concentration of the substance in the subject's interstitial fluid can be carried out repeatedly over a period of time.

This means that inconvenience and discomfort to the user is significantly reduced and moreover that tests can be carried out at regular intervals and at a greater frequency than would otherwise be tolerable. The result of this in the preferred application of the disclosure to blood glucose monitoring is that an improved insight into glycaemic control and the effects of food, medication and general trends on the glucose level is given. Moreover, by attaching the device to the patient and therefore making the device portable, it allows the patient to lead as normal life as possible. It also allows for the possibility of continuous measurements during periods when it may not be convenient to self-monitor by conventional manual methods of testing, for example during prolonged periods of exercise or whilst sleeping. The device could therefore include an alarm or means to activate an alarm in order to alert or wake the patient or any third party in case of a particular analyte level, for example in the case of hypoglycaemia.

Accordingly it is preferred that the devices comprise a penetration member with a sufficiently length substantially to penetrate the cutaneous layer of skin, most preferably integrally formed with the support member. Such devices comprise at least one microchannel for conveying the interstitial fluid to one or more of the sensing means.

Preferably, the device for measuring the concentration of glucose in interstitial fluid comprises means for attaching the device to the skin of a subject and a plurality of sensing means for making a plurality of measurements of said concentration over a period of time.

It will be seen that effectively this aspect of the disclosure provides a device which can perform a plurality of measurements *in situ*, that is without user intervention being required. This has clear benefits in removing some constraints on the number, frequency and regularity with which measurements can be performed. Each sensing element in the device is designed to be used for a certain predetermined time during which the signal will not significantly drift over time. In this sense " significantly " represents the amount of drift permitted such the measurement result would not be affected by an clinically unacceptable amount. After such a period fluid is then switched to a new sensor and the process repeated. Preferably the device is provided with flow control means for influencing the flow of fluid to the sensing means. Any suitable flow control method may be employed with corresponding means to affect such a control method. For example Piezo-electric pumping, electrokinetic or mechanical methods such as 'unblocking' the flow along a selected conduit. - e.g. by allowing a gas bubble to escape or by opening a valve.

The flow control means comprises a hydrophobic gate situated in the conduit/microchannel. A hydrophobic gate as herein disclosed refers to a hydrophobic surface region within a hydrophilic channel such that the flow of fluid is interrupted. By changing the hydrophobic nature of the gate, i.e. by making the hydrophobic region more hydrophilic, fluid may then be allowed to flow along the channel. Hydrophobic gates may be used to control the flow of fluid within a single microchannel or may be used to switch or redirect flow from one microchannel to another.

Alternatively, the hydrophobic nature of the gate may be maintained as it is and a increased pumping force (e.g. provided by a mechanical or electro-osmotic pump) may be applied in order for the fluid to breach the hydrophobic gate.

Preferably the device is arranged to direct fluid sequentially to each of the sensing means. The timing of the direction of the fluid to the sensing means could be pre-configured for example by software within the meter, such that the fluid is switched after a predetermined period of time. Preferably however the device comprises, or is adapted to interface with, control means to control said direction of the fluid to the sensing means. Such control is preferably automatic.

Additionally or alternatively however the control means may be such as to allow a user to specify when a measurement is to be made. This is beneficial as it allows measurement on demand which is useful for example in the case of blood glucose monitoring, as it allows the user to determine the effect on blood glucose of eating a particular snack or to determine how much insulin it is necessary to inject prior to eating or the user may simply want to carry out a check for reassurance. This enables the device to make periodic measurements of fluid from the body of the subject using fresh fluid for each measurement, thereby facilitating the desired object of monitoring the concentration of the substance in question over a period of time.

Where, as is preferred, each microchannel or other conduit is associated with a respective flow control means, each may be individually addressable by a suitable control means. This gives significant flexibility in how such a device may be used.

Preferably the device comprises a common fluid collection region in fluid communication with the bodily fluid to be measured - e.g. via a needle - each sensing means preferably being in selective fluid communication with said common collection region. Thus when viewed from a further aspect the present invention provides a device for making a plurality of measurements of the concentration of an analyte in a fluid comprising a common sample collection site in fluid communication with the fluid to be measured and a plurality of sensing means for measuring said concentration.

Microchannels as defined herein are provided for conveying the fluid to be measured to the sensing means. Furthermore in the context of a plurality of analyte sensing means, the provision of microchannels allows many test elements to be positioned in close proximity thereby enabling even devices comprising a large number of sensing means to be made relatively small. This is beneficial in applications where the size and weight of the device is an important consideration, such as when it is attached to the user's body.

As well as the understood benefits per se of a low sample volume arising from the use of microchannels, the Applicants have realised that such a small sample volume makes it practicable to change the mode of testing. More particularly it has been realised that a very low sample volume means that rather than conduct the usual reaction rate measurement as in known electrochemical devices e.g. for detecting blood analytes such as glucose, where electron transfer is measured as a function of time to determine the rate of transfer, an end-point test can be carried out in which the total amount of analyte in the sample volume is measured, thereby consuming substantially all of the analyte.

It has been appreciated that this is advantageous over rate measurements since the latter tend to be prone to interference, temperature and Haematocrit fluctuations (in the case of using blood). Whereas such a measurement method would take a prohibitively long time with known measuring devices, by using microchannels in accordance with the invention to give a required sample volume of the order of a few nano-litres, such a measurement can typically be completed in the order of a few seconds, thereby making it a practical proposition.

Preferably, the device is an electrochemical device comprising a sensor electrode disposed within a microchannel and means for measuring an aggregate currant passed by said electrode in use to give an indication of said amount of analyte in the sample.

The disclosure also extends to an equivalent arrangement using a non-electrochemical sensing means - e.g. a colorimetric one.

Any suitable transfer means for transferring the fluid from the user's body to the device may be employed. Preferably methods such as suction ultra-sound or iontophoresis may be used. More preferably however the transfer means comprises a needle. In particularly preferred embodiments the needle is a microneedle as defined hereinabove.

The needle is preferably shaped to aid skin penetration. For example the tip region of the needle is preferably substantially conical. Furthermore it is preferred that the tip region has a reduced cross-section - preferably less than 0.2mm in width, most preferably less than 0.05mm in width.

Moreover the needle is preferably arranged to minimise the risk of blockage upon insertion into skin. For example the aperture of the needle may be provided on a side surface of the needles, rather than at the tip as is conventional. Preferably the aperture of the needle is recessed, thereby avoiding contact with the skin upon penetration and thus potential blocking and/or damage

The needle preferably has a bore such that the sample fluid is drawn up by capillary action. Suitable needle bore sizes range preferably from 21-30 most Preferably 25. Any suitable inert and biocompatible material for the needle may be employed.

Examples of such inert materials include but are not limited to stainless steel, gold, platinum and metal-coated plastics

Most preferably one or more microneedles is employed as defined hereinabove.

The methods above may be supplemented by applying pressure to the user's skin around the site at which it is penetrated. Such pressure could be applied purely manually, but preferably the device comprises means - e.g. suitably configured resilient means - to apply the pressure. The device may have skin pressurising means as explained in more detail below and as shown in the figures. Examples of skin pressurising means are set out in copending application US09/877,514 filed June 8, 2001.

Preferably therefore the devices of the preferred examples comprise display means to display the concentration of the analyte being measured such as blood glucose. Such display means may be coupled directly to the measuring device, but preferably it is separate from the device and receives data therefrom by telemetry. This approach has the advantage that the measuring device can be very light and thus,comfortable to wear. For example the measuring device may be worn under clothing but monitored on a display means kept, say, in a pocket or for example in the form of a watch worn on the arm of the user, without the user having to disturb their clothing in order to view it.

The device may comprise or be coupled to means for administering a substance to a user on the device on the basis of the measured concentration. Thus in the previous example of a blood glucose monitoring device, rather than just being a passive monitoring device, undoubtedly useful per *se,* such a device can be used in conjunction with an insulin pump to maintain the user's glucose level within a desired range.

The insulin pump could be a separate device or alternatively could be integrated within the analyte sensing device per se. Controlling means present for example within the meter could control the amount of insulin administered by the insulin pump in response to the level of glucose measured by the device.

By effectively providing a feedback loop, preferred examples of this disclosed feature can allow a diabetic, to maintain control of his/her glucose levels with a minimum of intervention i.e. only to replace consumable items such as a sensor/insulin patch.

Furthermore, particularly where a continuous sensing means is employed, tighter control of, say glucose level, is achievable than where intermittent manual tests and insulin administration are used

Thus, in one example, the device of the present disclosure provides an apparatus for administering a substance to a user comprising a measuring device for measuring the concentration of an analyte in a bodily fluid from said user, said measuring device comprising a plurality of analyte sensing means and at least one conduit, preferably a microchannel, for conveying said bodily fluid to at least one of the sensing means; the apparatus further comprising means to administer said substance to said user on the basis of said measurement of concentration.

In accordance with all appropriate aspects of the disclosure, the measuring device preferably comprises a means for attachment to the skin of a user, for example in the form of a self-adhesive patch. This can provide a secure but comfortable arrangement for use over prolonged periods of time, and can be relatively unobtrusive.

The means for administering the substance may be entirely separate from the measuring device or integrated therewith. Preferably such an integrated administering means comprises a reservoir on or in the measuring device for dispensing the substance. In a particular example the substance, such as insulin, is contained within a reservoir on an adhesive patch. The actual means for getting the substance from such a reservoir could comprises anything suitable such as a pressurised supply in conjunction with a flow control means such as a valve. Preferably however a pump is used. In a preferred example a single pump may be used both to administer the substance, such as insulin, to a user's body and also to draw out blood or preferably interstitial fluid, to a sensing means for making an analyte concentration measurement e.g. of glucose.

Thus, for example, a suitable device may comprise an adhesive patch comprising a microneedle (as defined herein) coupled to or in fluid communication with an array of microchannels and a separate needle for injecting insulin. In a preferred example a single pump, e.g. a silicon micro-pump, may be used to inject insulin from a reservoir on the patch and to draw interstitial fluid over a glucose sensing means to a waste reservoir.

Where an example of the disclosure calls for control and/or data processing means, these will generally comprise electronic means such as an integrated circuit or the like. A power supply is then also required. Preferably such control/processing means are portable. It or they may be provided in an integral package with the device e.g. the adhesive patch. Alternatively the control/processing means and/or power source may be provided separately and communicate with measuring device via a wire or wireless telemetry link as mentioned above.

The power source may be a battery or may be a 'renewable' source such as a solar cell or a dynamo energised by movement of the user. Of course a combination of these could be employed.

Measuring devices in accordance with the present disclosure may be fabricated using any suitable technique. In particular, where provided, the microchannels may be made using any suitable micro-fabrication technique such as but not limited to embossing, plasma etching or injection moulding

In one preferred example, electrodes are provided on opposing sides of a fluid channel by forming a first channel which is filled with a conductive material, and forming a second channel for conveying the test fluid, the second channel cutting across the first thereby thereby forming two conductive portions within respective opposite sides of the second channel.

The conductive portions formed in accordance with the disclosure could be utilised for an electrochemical sensor arrangement. Micromachining techniques for making the abovementioned intersecting channels are preferred since they can be used to fabricate microchannels which can be formed close to one another, permitting dense arrays thereof. Furthermore, where the device has flow control means operating via electro-osmotic force, the driving electrodes are preferably positioned in close proximity to one another. This allows high electric fields to be achieved without applying unnecessarily high voltages. Preferably such driving electrodes are provided substantially on one side of a channel. In one embodiment the driving electrodes extend around the wall of the channel.

In a preferred variant of the method above, one or more electrodes may be formed on a second substrate which is then laminated to the main support member of the device. Methods used to deposit the electrodes onto the second substrate may be chosen preferably from a printing method, more preferably a screen-printing method. Alternatively, chemical or physical vapour deposition techniques could be employed. Generally speaking, the electrodes according to all examples of the disclosure may be formed of any suitable inert material such as carbon, gold, platinum, etc. According to one example, carbon electrodes, optionally coated with reagents are provided on the second substrate by screen-printing, which is then laminated onto the support member thus closing the channel or channels and two gold electrodes are provided adjacent one another on a substrate laminated onto the support member for an electro-osmotic pump.

If a second substrate is provided, preferably it is arranged to close the channel provided on the support member. This allows a very straightforward fabrication method in which electrodes are formed within a closed channel.

Lamination of one substrate to another will normally be carried out such that both laminates are perfectly aligned and that no further trimming or cutting is necessary. However, the device could be fabricated for example by firstly a lamination step followed by a cutting step whereby the second substrate may be trimmed to the shape of the support member. Lamination may be carried out by various methods such as ultrasonic or thermal welding or bonding, or by the use of an adhesive.

According to one example, the support member is formed with an integral needle at one end and the second substrate is then laminated onto the support forming a channel and leaving the penetration member exposed. According to another example the integrated skin penetration member is provided is open on one side. The penetration member is arranged so that upon insertion into skin, the skin itself effectively forms a wall of the member to that it can act like a hollow needle. Most preferably this is achieved by forming the penetration member with walls tapering away from the open side - e.g. a V shape. Thus the device may comprise a skin penetration member having at least one longitudinal side open, the other sides being arranged so as effectively to cause the penetrated skin to act as the remaining longitudinal side of the member when the penetration member is inserted into the skin.

Where an optical measurement technique is employed, as an alternative to an electrochemical measurement technique a light sensing means will, in general, be required. In some cases a light source may also be required, but is not always the case, for example in the case of chemiluminescent measurement. Any such light sensing means and/or the light source may be provided integrally with the non-disposable measuring device e.g. a test-meter. According to one example it or they are provided separately of the part of the device which is brought into contact with the sample fluid - e.g. a skin patch. This means that the device itself can be made disposable while the relatively more expensive light sensing means and associated electronics for example could be provided in a separate test meter.

In preferred examples the test device comprises means for optimising the light transfer from the sensing means to the optically sensitive means. In a simple example such means comprises a lens, e.g. integrally moulded as part of the support member for the test device. Additionally or alternatively the device may be arranged such that the light sensitive means views the sensing means along the conduit, e.g. microchannel, along which the sample fluid passes. In other words the conduit, preferably a microchannel, acts as a light pipe. By measuring light transmission or reflectance along the microchannel rather than across it, the path length and therefore the optical density may be increased for a minimal sample volume, thereby making its measurement easier and more accurate. The material from which the conduit is formed is preferably chosen so to maximise light throughput at the frequencies of interest.

It will be appreciated by those skilled in the art that the arrangement described above is beneficial in its own right in enhancing the signal that may be measured from a minimal sample volume and thus when viewed from a yet further aspect the present disclosure provides an apparatus for measuring the light from an assay comprising an elongate conduit portion along which a sample fluid is drawn in use and a light sensitive means arranged to be sensitive to light coming substantially from the longitudinal axis of said conduit portion.

In one particularly preferred example a disposable skin patch is provided with a moulded plastics lens over the analyte sensing means. A corresponding test meter is designed to be placed over the patch and comprises a light sensitive element which sits over the lens when the meter is placed over the patch.

### III. Brief Description of the Drawings

Certain preferred examples of devices will now be described, by way of example only, with reference to the accompanying drawings in which:
Figure 1 shows a first example of a device, in the form of a skin patch, in cross section;
Figure 2a shows an alternative microchannel arrangement;
Figure 2b shows a multiple channel/sensor arrangement;
Figure 3 depicts a cross section view of the skin patch of Figures 1, and 2 attached to the skin of the user with a controller unit attached to the skin patch;
Figure 4 depicts a display unit;
Figure 5 depicts another example of a device, showing schematically a skin patch integrated with a needle, which also acts as an electrode, in cross section;
Figure 6a depicts a microchannel and optochemical sensor in plan view;
Figure 6b depicts a microchannel and electrochemical sensor in plan view;
Figures 6c to e are cross sections of the microchannel of Figure 6b with fluid progressively entering the channel;
Figure 8a shows a further example of a device, in the form of a single-use device with an integrated puncturing means;
Figure 8b is a cross section through a user's skin having the device of Figure 8a therein;
Figures 8c and 8d depict the construction of a device similar to that in Figure 8a;
Figure 8e is an alternative embodiment, shown in perspective view, of a puncturing means;
Figure 8f is a still further embodiment of an alternative puncturing means shown in perspective view;
Figure 8g is a side-sectional view of the example of a device of Figure 8f taken along line X - X in Fig. 8f;
Figure 8h is a cross-sectional view of the lance of Fig. 8e in tissue;
Figure 8i shows an integrally formed base member and lance showing a vent and a capillary sensing channel, but with upper laminate removed for clarity;
Figure 12a is a perspective view of a microchannel,
Figure 12b is a close-up, partial, perspective view of a microchannel; whereby electrodes are provided on opposing sides of a fluid channel by forming a first channel which is filled with a conductive material, and forming a second channel for conveying the test fluid, the second channel cutting across the first thereby thereby forming two conductive portions within respective opposite sides of the second channel.
Figure 17a is top view, taken in perspective, of a first preferred example of a chip for extracting and distributing a fluid sample and having a meander shaped waste reservoir;
Figure 17b is the view of the device of Figure 17a modified to illustrate a column shaped waste reservoir;
Figure 18 is a bottom view, taken in perspective, of the chip of Figure 17a;
Figure 19 is a plan view of a measurement channel of the chip of Figure 17a;
Figure 20a is a plan view of the meander shaped waste reservoir of the device of Figure 17a;
Figure 20b is a plan view of the column shaped waste reservoir of the embodiment of Figure 17b; and
Figure 21 is a plan view of the switching mechanism shown in Figure 20a.

### IV. Detailed Description of the Drawings

### A. Skin Patch - General Description

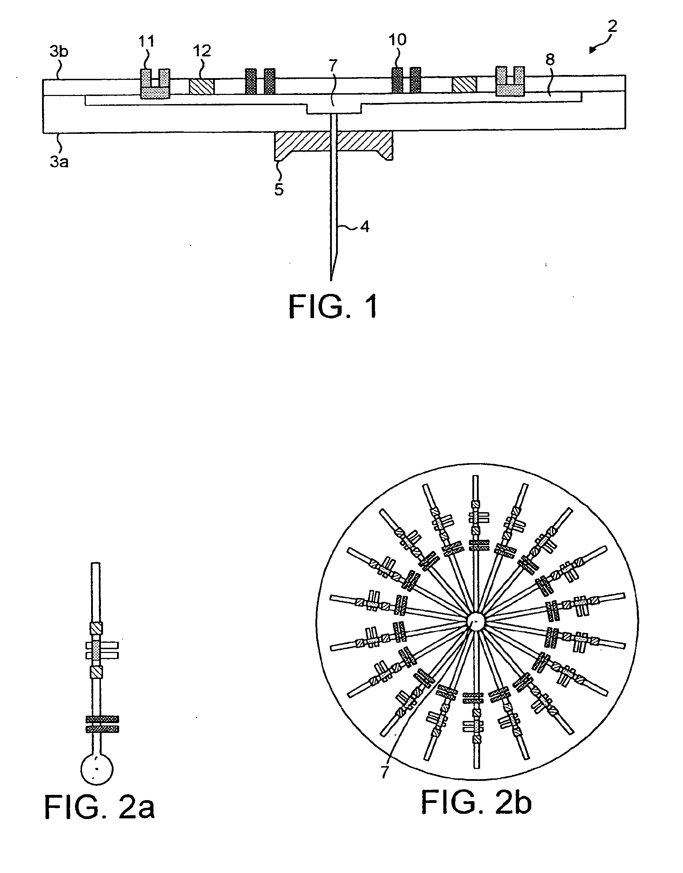

Turning to Figure 1 there is shown a skin patch 2 suitable for measuring the level of blood glucose in a user. Patch 2 is made up of two layers 3a and 3b

The lowermost layer 3a is made of an suitable microfabricated plastic such as polyester, polycarbonate, polystyrene, polyimide, or other suitable microfabricatable polymers of suitable dimensions to allow it to be worn comfortably for a prolonged period of time, and optionally has an adhesive on its underside to allow the patch to be securely attached to the skin of a user. An optional pressure ring (5) is designed to apply pressure to the surface of the skin to enhance the flow of fluid from the body of the patient into the device. The pressure means may also be integrally formed and of the same material as that of 3a. The drawing is not to scale and thus the various features may be of a different relative thickness dimension than illustrated. It is understood that figure 1 is shown for illustrative purposes only and as such the underside of 3a may be of any shape that would facilitate fixing of the device to the skin, for example when the pressure means is incorporated. The example is not limited as such to a pressure ring and other types of pressure extraction designs could be used. A penetration device (4) e.g. a needle, lancet or cannula is attached to the lowermost layer 3a, through which fluid passes into the collection and fluid collection and distribution port (7) formed in the upper surface of layer 3a. Layer 3a has on its upper surface microfabricated channels. Port (7) is also formed by the same microfabrication process as the microchannels.

Laminated to the lowermost layer is a substrate layer 3b, preferably formed of the same material as that of the lowermost layer. This serves to close the microchannel. The microchannel and penetration member may optionally be coated with a hydrophilic material and/or an anti-coagulant such as a heparin attached to the inner surface such that during use it will not diffuse away. Also shown is the microchannel system 8, corresponding electrochemical detectors 11 and optionally the electro-osmotic pump systems 10 and the hydrophobic gates 12. Each of the electrochemical detectors 11 and electro-osmotic pump systems 10 are electrically connected via conductive tracks (not shown) to respective terminals (not shown) at the edge of the patch. Not shown in Figure 1 are the fluid reservoirs. Fig 2a shows another device whereby hydrophobic gates are present on either side of the sensor.

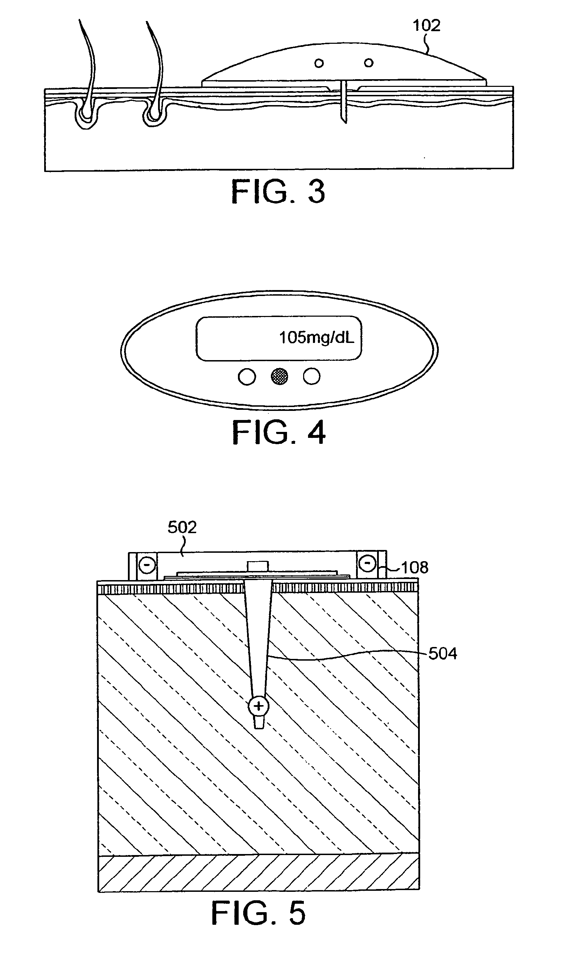

With respect to Figure 3, a controller unit 102 is brought into contact with these terminals so as to make electrical contact in use in order to transmit the measurement output signals between the patch and a display unit 103 (see Figures 3 and 4).

A control unit 102 for the patch 2 is shown in Figure 3. As may be seen, this sits on top of the patch 2 and is secured to upper side of the patch in a suitable manner(not shown). The control unit serves to control the fluid pumping mechanism, hydrophobic gates, fluid switching means and analyte sensors. The control unit is also provided with means such that radio frequency communication may be made, for example with the meter, for transmission and receipt of data. The control unit would also determine when all the microchannel/sensors had been used and transmit a signal accordingly. The control unit would also have means to detect any malfunctioning of the device and to also transmit this information.

The control unit 102 is preferably battery operated and is capable of transmitting signals to a wireless display unit (Figure 4) for example a radio frequency communication.

Figure 2b shows a multichannel arrangement with the proximal end of the needle 4 is in fluid communication with a fluid collection and distribution port (7) formed in the substrate layer 3b of the patch. Radiating out from the manifold 6 are eighteen of the microchannel systems 8. Not shown is the fluid switching means.

### B. Micro-Channel System with Flow Control

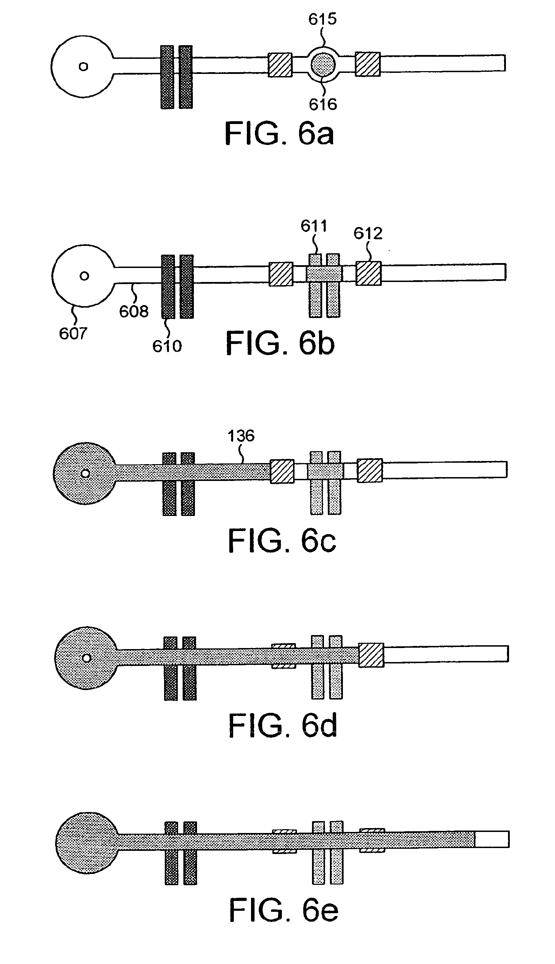

One such microchannel system 8 is shown in greater detail in Figure 6b. The microchannel 608 is in direct communication with the port 607 into which the fluid flows from the needle 4. Downstream of the port 607 is an electrode system comprising a pair of inert electrodes 610 providing electrosmotic flow. The electrodes are formed on a second substrate layer 3b (omitted from this figure for clarity). The second substrate layer is then laminated onto the first substrate layer 3b thereby closing the microchannel 126 and bringing the electrodes 610 into contact with it. The electrodes 610 are positioned adjacent to each other, together forming an electro-osmotic pump system. By applying a voltage difference across the electrodes, an electric field is generated which drives fluid along the microchannel 608.

Positioned along the microchannel 608 is one or more hydrophobic gates 612 which prevents the flow of fluid by capillary action along the microchannel. Downstream of the hydrophobic gate 128 is an electrochemical sensor 611 comprising at least two electrodes also formed on the upper substrate layer 3b. At least one further hydrophobic gate similar to the first, is optionally provided downstream of the sensor 12. This serves to stop the flow of fluid past the electrodes to allow the possibility for an end-point detection of the analyte.
It will be understood by those skilled in the art that the hydrophobic gates may be constructed of any suitable material, including, but not limited to, PTFE, polycarbonate, polyisobutelene, PMMA, dodecyl acetate, silicon rubber, synthetic way, octadocyl mercaptane, dodecyl mercaptane, and/or octa decyltrichloro silane.

### C. Skin Patch Operation

Operation of the skin patch 2 will now be described. The patch is attached to the skin of a user causing the needle 4 to penetrate substantially into the cutaneous layer of skin. Cutaneous fluid is drawn up the needle 4 and into the port 7 of the device. Referring now to Figure 6c it will be seen that the fluid 136 is drawn into the microchannel 126 as far as the hydrophobic gate 128 where its flow is arrested. When a measurement is required, the controller unit 102 issues an appropriate signal to the electro-osmotic pump 610, in the form of a voltage difference across the two electrodes. This causes the fluid 136 to flow through the hydrophobic gate 128 and past the analyte sensor 611 until it reaches the second (optional) gate. Once the interstitial fluid 136 comes into contact with the sensor 12, a measurement is performed by the analyte sensors.

The controller unit 102 is provided with control means or means to initiate the flow of interstitial fluid sequentially to each of the microchannels (8) after a predetermined period of time. Once all the tests on the patch 2 have been performed, the controller 102 transmits a signal to the display means 103 to alert the user so that the patch 2 can be discarded and a fresh one applied.

### D. Alternative Example with Optochemical Sensing

An alternative form of microchannel 126 is shown in Fig. 6a. This is similar to that shown in Fig. 6b except that the electrochemical sensor 611 is replaced by an optochemical one 615. The optical sensor comprises a reaction chamber 616 which for example contains Glucose oxidase (GOD), Horseradish Peroxidase (POD) and a leuco-dye, (a colourless precursor of the dye molecule). (e.g. 2,2-Azino-di-[3-ethylbenzthiazoline-sulfonate]
The optical system is not limited to the above example and could comprise any suitable enzyme-dye combinations. The reaction which takes place is as below:

It will be seen from the above that the dye changes 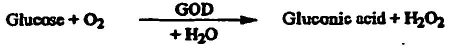 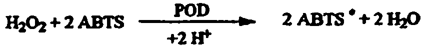 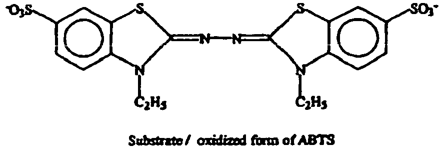 colour in accordance with the amount of glucose in the sample. In order to measure this, the chamber 129 has an optically transparent upper surface. This arranged is to be aligned with the tip of an optical fibre in a modified control unit (not shown) so that the fibre is approximately 2 to 3 mm above the dye spot. The other end of the fibre is in optical contact with a light source and light sensitive diode sensor which is sensitive to a particular wavelength of light (for example 438nm in the case of ABTS) emitted by the transformed dye. The degree of absorption therefore gives a measure of the amount of transformed dye and thus the amount of glucose present.

As illustrated above, glucose present in the fluid acts as a substrate for GOD, which breaks the glucose down into glucono-1,5-lactone and hydrogen peroxide. POD subsequently catalyses the oxidation of the leuco-dye using the hydrogen peroxide, resulting in the production of a coloured dye which is subsequently detected. Those skilled in the art will understand that instead of 2,2-Azino-di-[3-ethylbenzthiazoline-sulfonate], any suitable leuco-dye may be used, e.g. Tetramethylbenzidine-Hydrochloride, or 3-Methyl-2-Benzothiazoline-Hydrazone in conjunction with 3-Dimethylamino-Benzoicacide.

The microchannels described as hereinbefore use electrochemical or colorimetric means to detect glucose. The skilled person would understand that other detection means, such as infra red detection, filter photometry or Kromoscopy may be used.

### F. Electrode Penetration Member

Figure 5 shows schematically a further preferred example of a device. Similar construction to that of the previous embodiments and thus it has a hollow needle 106, for example but not limited to 1.4mm long and 0.3mm wide which penetrates substantially the cutaneous layer of the skin 113 of the user.

In this embodiment however the needle 106 also acts as an electrode which serves to extract fluid from the skin. At least one electrode 108 is provided such it makes contact with the skin. As shown in Fig.5, the needle is poised at a positive potential. By applying a voltage difference across the electrodes 106 and 108 an electric field is generated along the needle 106 and manifold 111 which stimulates the skin and increases the perfusion of interstitial fluid out of the skin.

### G. Alternative Penetration Member Construction

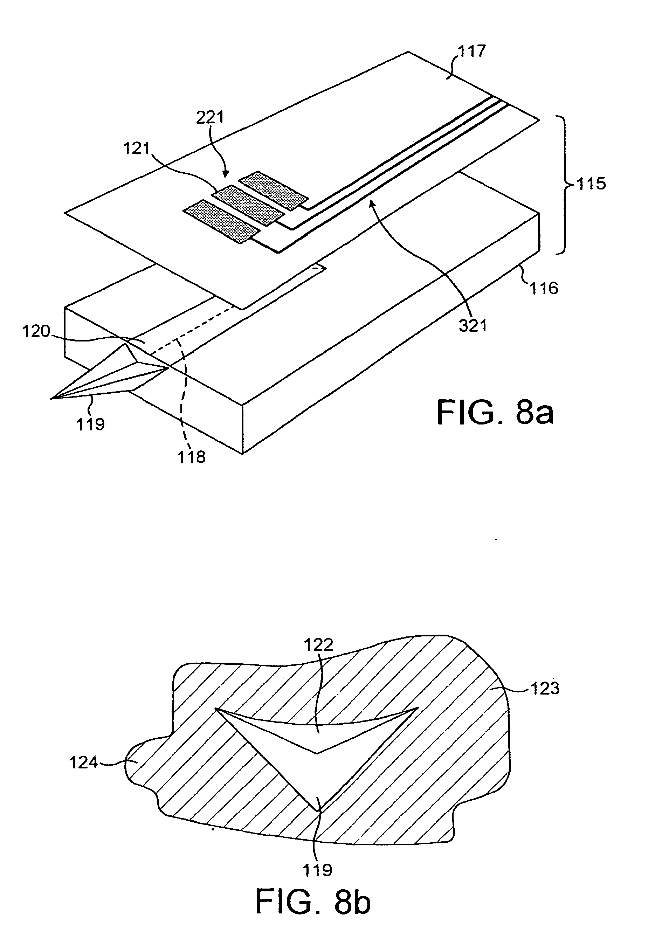

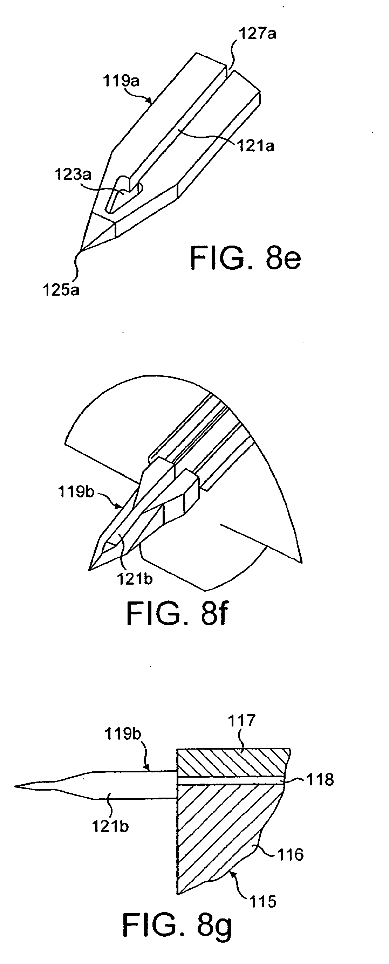

Figures 8a to 8d show two further examples of devices. In contrast with the foregoing examples, these are both single-use strips suitable for measuring blood glucose of a user. They are therefore used in a similar way to conventional test strips. However a principle difference is that each has an integrated lance 119 at one end.

Considering the device 115 shown in Fig 8a, it will be seen that it is made essentially of a layer 116 onto which a second layer is attached or laminated (not shown). The lowermost substrate layer 116 comprises a moulded or stamped microchannel 118, as well as the integrally formed lance 119 arranged in close proximity with the entrance 103 of the microchannel. During manufacture, the microchannel 118 maybe coated with suitable reagents, such as glucose oxidase in the case of glucose detection, which can be applied by any conventional means, such as printing for example screen-printing or ink-jet printing, or spray coating during manufacture. Alternatively, the microchannel may be free of enzyme, the reagents being provided on the electrode system (121) located on the underside of the uppermost layer (117).

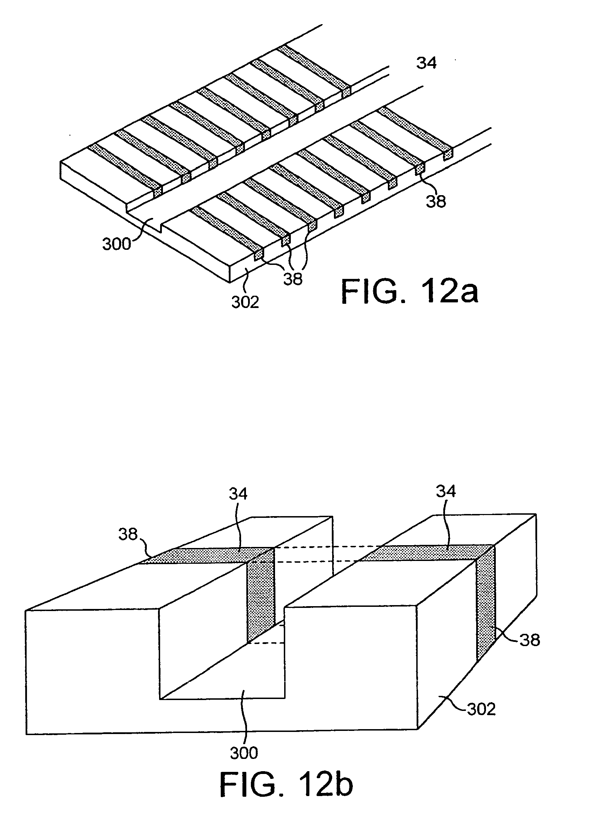

The uppermost layer 117 may also be a conventional biosensor test-strip which could be attached to the lower surface such that the electrodes are positioned on the underside of the formed channel. This particular electrode system 121 comprises three electrodes 221 of which at least one is covered with layers of enzyme and electron mediator such as glucose oxidase and ferricyanide to form a working electrode for detecting glucose, as is well known in the art, the other two being a counter and reference electrode. The electrode system may comprise two working electrodes which serve to compare currents at each and measure the channel filling speed of the blood or interstitial fluid. Alternatively the electrode system could comprise just two electrodes, a working and counter/reference. Not shown in Figures 1-21 is a venting device such that air may be displaced upon uptake of fluid into the microchannel. A vent or vents may be provided at any convenient location. Corresponding tracks 321 allow electrical connection to be made to the electrodes at the distal end of the strip when it has been inserted into a suitable test meter. The upper layer 117 may be slightly longer than the lower one 116 to allow access to the tracks 321 for this purpose.

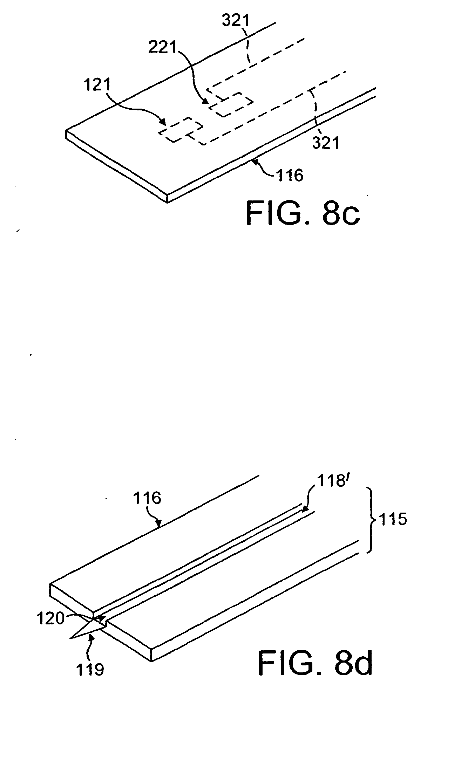

It will be noticed in particular that the lance 119 of the strip 115 in Fig. 8a is essentially V-shaped in cross section and tapers towards its tip. This means that when it is used to puncture a user's skin 123, as is shown in Figure 8b, the two sides of the V force back a portion of the skin 123, forcing the epidermis to form the remaining wall 123 of an enclosed channel 124. Thus an open channel is effectively transformed into a closed one when it is inserted into skin. This allows fluid to be drawn up the channel 124 so formed and into the microchannel 118, without having to mould a very fine hollow needle. The microchannel 118 may also be formed with a V-shaped profile for convenience of fabrication, but this is not essential as may be seen from the slightly modified embodiment of Figs.8c and 8d in which the microchannel 118' has a rectangular profile.

In the use of the strip 115, the user pierces their skin with the lance 119 and interstitial fluid or blood is made to flow, by means of capillary action, through channel 124 formed by the lance (119) and the skin 122, into the microchannel 118 and thus over the electrochemical detector 121.

The user then withdraws the strip 115 and inserts the opposite end into a conventional style test meter which makes electrical connection to the three conductive tracks 321. Preferably however, the meter is an integral part of the lancing system such that the user does not need to insert the strip into the meter and is automatically provided with the measurement result.

Figures 8e through 8h show alternative examples of lances for penetrating into a body-fluid laden layer of skin as an alternative to the example of the lance 119 of Figs. 8a and 8b. In Figure 8e, the lance 119a is an integrally formed pointed protrusion from the device 115 (not shown in Figs. 8e but identical to that in Fig. 8a) with a longitudinal capillary channel 121a cut completely through the thickness of the lance 119a. At a pointed distal tip 125a of the lance 119a, the lance 119a is provided with an enlarged area 123a of the channel 121a. The enlarged area 123a also is cut completely through the thickness of the lance 119a. At its proximal end 127a, the capillary channel connects with the microchannel 118 of the device 115 of Fig. 8a.

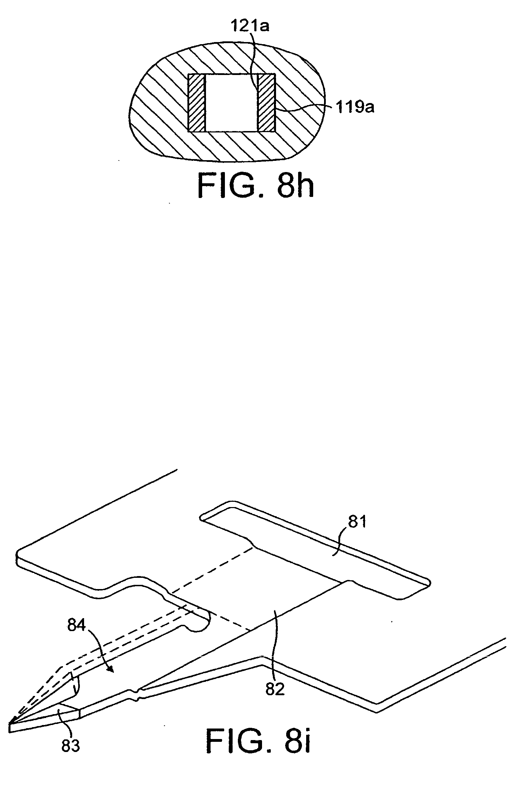

As shown best in Fig. 8h, the example of Fig. 8e permits fluid to enter into the capillary channel 121a from opposite sides of the lance 119a and with the wall of the skin cooperating with the walls of the lance 119a to define an enclosed channel 121a. Fluid then can accumulate in the pooling area 123a and flow from the pooling area 123a into the capillary channel 121a as well as flow directly from the skin into the capillary channel 121a for passage to the microchannel 118.

In the embodiment of Figure 8f, a lance 119b is of a design similar to that of Fig. 8e is shown but excluding the large pooling area 123a. Elimination of the pooling area 123a permits a narrower transverse dimension to the lance 119b.
In addition to fabricating the device from molded parts, the base member 116 and lances 119, 119a, 119b can be stamped from electrically conductive material. In such cases, the base member may be an electrode. An electrically conductive base member and lance can be stamped from metal as described or formed in any other acceptable manner (e.g., photochemically etching a metal stock material, machining or other fabricating technique). While the electrically conductive base member can be made of stainless steel it can be also be plated with a second metal such as for example gold , platinum or silver. The electrically conductive base material may also be used as a counter electrode.

Fig 8i shows an integrally formed base member and lance stamped from preferably one piece of sheet metal. The metal is preferably, but not limited to, stainless steel optionally coated with a noble metal such as gold or silver. Also shown on the base sheet is a microchannel 84 onto which a second layer such as a test-strip could be attached. It is intended that such a device as illustrated in Fig 8i would be incorporated into a lancet firing device such that the device could be fired into the skin. Furthermore the individual devices may be loaded and stored in a cassette comprising individually sealed pouches. Such a cassette could also be stored within the lancet firing device.

The stamped penetration member with a rectangular vent 81 which also serves as a capillary break ensuring that once fluid is taken up by the lance 83 into the sensing zone 82, the flow of fluid is halted. The vent may be of any suitable size or shape.

### J. Detailed Preferred Example

### 1.Sampling Module

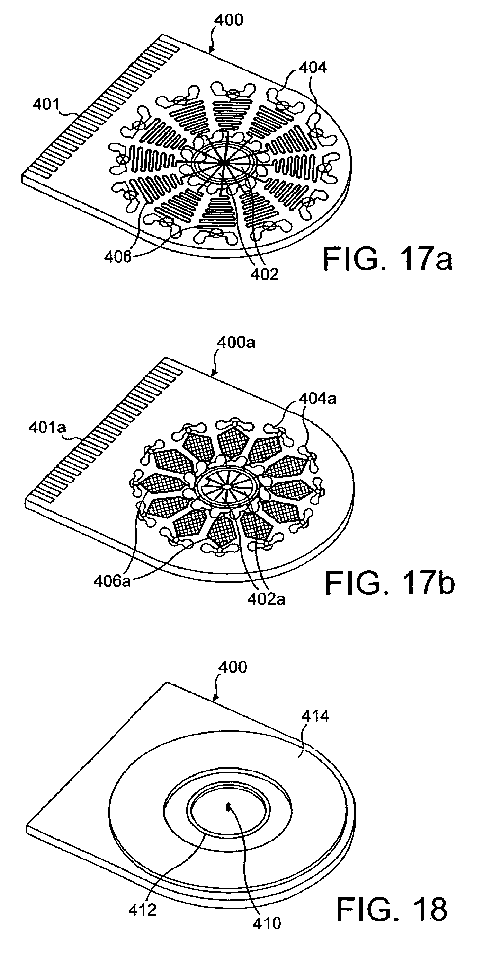

With reference now to Figures 17a - 21, the present disclosure will now be described with reference to a most preferred example of a device in an ex vivo continuous monitoring system.

The ex vivo continuous monitoring system consists of several major subsystems including sample extraction, sample fluid distribution, and electrochemical detection. As will be more fully described below, a body fluid sample can be extracted from the skin and guided into a channel for the electrochemical determination of glucose concentration.

After a predetermined period, the body fluid is directed into a different, previously unused channel. This process avoids false results otherwise arising from fouled electrodes or denaturant enzymes. Since the channels into which the fluid is re-directed are not filled with a sample solution or any other liquid, the electrochemical system in the channel will not show aging effects.

The disposable portion of the ex vivo continuous monitoring system is referred to herein as a chip. Figures 17a, 17b show embodiments of such a chip 400, 400a. In the embodiments of Figures 17a, 17b, each chip 400, 400a has for example, but not limited to, twelve measurement channels 402, 402a with switching mechanism 404 and 404a and waste reservoirs 406, 406a. Differences in the examples of chips 400, 400a will be described below with initial description being limited to a discussion of chip 400. It will be appreciated that chip 400a is identical to chip 400 except as will be described. Similarly elements are numbered similarly with the addition of an " a" to distinguish examples.

The chip 400 has an edge connector 401 for electrically connecting electrodes (as will be described) to a controller (not shown). A controller can contain logic, memory and processors for controlling the electronics of the chip 400 and optionally displaying any outputs (as, for example, the function and operation of controller 102 in Fig. 3).

Preferably the measurement results are transmitted by radio frequency by the controller to a remote control allowing the user to view the result and optionally to communicate or interact with the controller of the monitoring system.

The sampling chip 400 extracts body fluid from the skin. In the preferred example, the chip 400 extracts interstitial fluid (ISF) from substantially the cutaneous layer of a patient. With reference to Fig. 18, the chip 400 includes a suitable cannula or needle 410, a spring-: loaded hub 412 to pressurize the skin and means such as an adhesive 414 to fix the chip 400 to the skin. Sampling modules with needles and spring-loaded hubs are shown in U.S. Pat. Nos. 6,203,504 and 5,746,217. It will be appreciated the foregoing description is a preferred example. Any technique for obtaining a sample of body fluid may be used in combination with the teachings of the present disclosure.

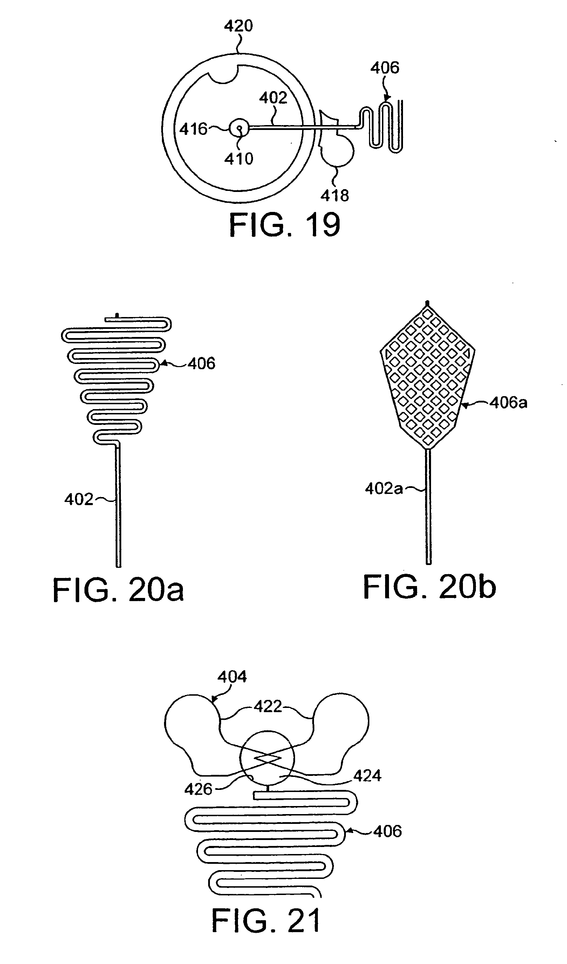

Body fluid enters the needle 410 due to the pressure applied by the hub 412 and travels in to the micro-channels 402 as described above with reference to Figs. 1a, 1b and 2. The needle 410 discharges the body fluid into a common distribution depression 416 shown best in Fig. 19). The distribution depression 416 is in fluid flow with the micro-channels 402. In the embodiment shown (having 12 channels of the dimensions described, the shape and the volume to the distribution depression 416 is preferably a disk shaped void with 1 mm diameter and 100 µm depth. These dimensions and shape may vary depending upon the size, number and flow rate of individual channels. Not shown in the drawings, a portion of the micro-channel 402 between the distribution depression 416 and a ground or reference electrode 420 may be restricted in size to reduce flow rate variability and therefore control the rate of flow.

Means may be provided within the device for the removal of undesirable gas-bubbles from the fluid sample. Such means could comprise a filter positioned between the needle and the common reservoir.

### 2. Continuous Electrochemical Detection of Glucose

The electrochemical detection system consists of an electrode system shown best in Fig. 19. The electrode system includes at least one working electrode 418 (by way of non-limiting example, gold or carbon) and a reference electrode 420 (e.g., silver/silver-chloride). The reference electrode 420 is circular to serve as a common electrode for each channel 402, thus reducing the number of contacts necessary. Alternatively, each channel can be provided with a unique reference electrode. Each channel 402 has a unique dedicated working electrode 418. In addition, a counter electrode (not shown) may be added. The electrodes 418, 420 are disposed to contact fluid in the channel 402. As previously described, the channels 402 are formed as open channels in a substrate layer. The electrodes 420, 418 are screen-printed on an overlying laminate layer which also serves to seal the channels 402. The working electrode material is dependent on the enzyme ink being used. For example, one can distinguish between a redox-mediated system for the carbon working electrodes and an oxygen mediated system for the gold or platinum electrodes.

While a specific electro-chemical system will now be described, it will be appreciated that such description is illustrative and any suitable system could be used.

In the case of the redox-system, an organo-metal-complex (which is linked to polymers forming the ink body) acts as a conductor and electron acceptor for the enzyme. The electrons are transported by the redox-mediator directly or by an electron hopping mechanism from redox-center to redox-center to the working electrode. At the working electrode, the mediator is finally recycled to its original redox-state before it can react with the enzyme protein in a new cycle. The following are suitable for this detection method: glucose oxidase from aspergillus niger (GOD EC 1.1.3.4) and PQQ dependent glucose dehydrogenase (GDH EC 1.1.99.17).

The enzyme ink is a cross-linked hydrophilic gel allowing the penetration of sample fluid but restricting the movement of the enzyme thus it is fixed to the electrode. This is an important property of the enzyme ink because the concentration of the enzyme has an effect upon the total response of the sensor. The same effect can be observed with the redox-mediator, due to the small molecular weight it cannot be entrapped in the same way as enzymes. Therefore, in the preferred embodiment, it is preferred to link the redox-mediator molecule to the large molecules such as the enzymes, proteins, or to the polymers of the ink material directly. In case of oxygen-mediated systems the situation is simpler. The enzyme ink needs only to contain enzyme as active component, the oxygen itself is dissolved in the sample fluid and is transferred to the sensor from the sample stream in the same instance as glucose.

In case of glucose determination the oxygen-mediated system can be created with GOD based on the ability of the enzyme to build hydrogen peroxide from oxygen and glucose. 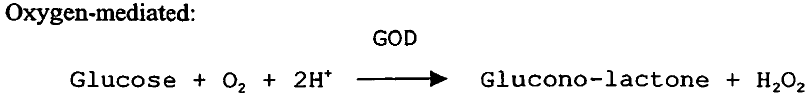 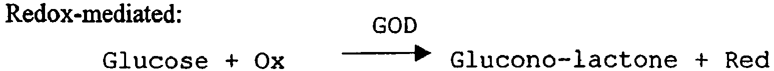 Ox = oxidized mediator species e.g. K₃[Fe(CN)₆], p-benzoquinon, Ferrocinium
Red = reduced mediator species e.g. K₄[Fe(CN)₆)], hydroquinone, Ferrocene

Hydrogen peroxide is a very reactive molecule, which can function as mediator to transport electrons for the enzyme to the electrode surface.

Oxidation of hydrogen peroxide on gold:

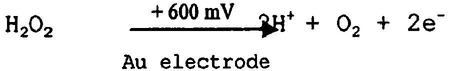

Reduction of hydrogen peroxide on gold:

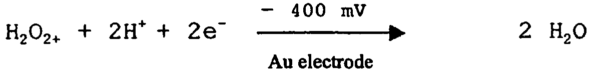

Normally only the oxidation of hydrogen peroxide is used for analytical purposes due to the possibility of direct oxygen reduction on negative polarized noble metal electrode during the reduction of hydrogen peroxide. However, the reduction of hydrogen peroxide would open the possibility to use more cost effective electrode materials such as silver instead of gold, platinum, or palladium. In comparison to the noble metal electrodes we need for the oxidation or reduction of hydrogen peroxide carbon electrodes can be used for the oxidation of the redox-mediator.

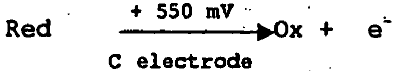

In the preferred example, the electrodes are deposited on a film such as polystyrene or polycarbonate of about 10 - 200 µm and, more preferably, about 30 to 75 µm) using screen-printing technology. Subsequently to this print step the working electrode is coated with ink containing enzyme and in the case of carbon working electrodes with an ink containing enzyme and mediator.

This coating step could be accomplished with the same printing methods as used for the electrode material. Thus the production could be done in one machine with different print heads or stations on a continuous web. Such a process is described in " Continuous Process for Manufacture of Disposable Electrochemical Sensor", US patent application Ser. No. 09/537599.

### 3. Fluidic Elements of Ex Vivo Continuous Monitoring

A schematic diagram of one measurement channel 402 is shown in Fig. 19. The channel 402 extents from the needle 410 and distribution depression 416 and through the main channel 402 (of dimensions for example of 200 µm x 100 µm) containing the electrodes 418, 420.

As previously described, a flow restricted portion of the channel 402 namely a small cross section (e.g., 30 µm x 30 µm) may be provided between the distribution depression 416 and the common electrode 420. Such a flow restriction levels out different flow rates during the extraction of the body fluid. These flow rate differences are caused due to the changing physiological conditions during the extraction. In general, one could observe a higher flow rate at the beginning of a sampling period compared to the extraction rate at the end. A thin capillary can counter-act this behavior.

The electrodes 418, 420 are not part of the injection molded base plate of the chip 400. Instead, they are placed on top of the open U-shaped or rectangular channel 402 of the base plate of the chip 400 in a separate lamination step.

Extending from the measurement channel 402 is the waste reservoir 406. The waste reservoir 406 stores the sample solution after its evaluation by the electrochemical sensing in channel 402. The size of the waste reservoir 406 defines the amount of time one channel can theoretically be used. After the reservoir 406 is filled, the controller can activate the next channel to continue with the detection of glucose in the body fluid of a patient.

It is important that the reservoir 406 is large enough to support more then a two-hour measurement. Thus, a chip 400 with twelve channels 402 can run for 24 to 28 hours allowing the patient a convenient change to a new chip (e.g. during his daily morning routines). However, as will be appreciated by those skilled in the art, reservoirs 406 larger then 5 µL (which is enough to support the flow for more then two hours with a flow rate of 30 nL/min) could allow longer runtimes. Thus, the entire chip 400 could last for more than one day. The number of channels in a device is not limited and may vary depending upon the measurement technique, the length of monitoring period and the size of the device and could exceed 100.

Figures 20a, 20b show two different representative examples of the waste reservoir 406, 406a. Waste reservoir 406 is a meander-shaped extension of the channel 402. While easy to manufacture, such a design can build up high back pressure in the system, due to the weaker capillary force, then the design of waste reservoir 406a and is not as space efficient.

The column filled reservoir 406a has advantages compared to the meander-shaped reservoir 406 regarding the size and the integration into a 12-channel chip 400a. It could be deeper than the reservoir 406 while producing a higher capillary force to take up the extracted and evaluated sample fluid from channel 402a. In principle, waste channel 406a resembles a plurality of capillaries with a cross-section for example of 400 µm x 400 µm compared to only one capillary with the same cross-section in the design of waste reservoir 406. The shape of the columns is not limited to simple squares. They could be formed as circles, pentagons, hexagons, octagons or the like. Concerning the flow characteristics inside a column field the hexagon shaped columns shown are the most preferred version. The size and geometry of the channels and reservoirs may vary.

The base plate of the chip 400 can be produced in a molding process, which is optimized for the small features and structures. A wide variation of plastics can be used for the base plate of the chip 400 (e.g. polystyrene, polycarbonate, polymethymethacrylate, polyester, and others). Preferred polymers are polycarbonates. These allow a subsequent laser finishing to generate a secondary micro- or nano-structure (e.g., any desired patterns or other finishing can be formed in the micro-channel). Polystyrene shows preferable characteristics in the lamination process. Thus polycarbonate could be used as the lower laminate and polystyrene used as the upper.

The chip 400 itself consists of three major part a) the chip base plate with all the fluidic elements described above, b) the electrochemical detection system screen printed on a polymer foil, and c) the sampling hub with extraction needle. These elements are described in the foregoing sections.

Normal lamination processes use foils coated with a pressure sensitive or hot melt adhesive to join a foil to a substrate or another foil. Such a standard process may present problems in conjunction with the described device. First, foil is needed which is suitable for the printing process. This is difficult with any pressure sensitive adhesive due to problems within the printing equipment. Such problems can be addressed with a foil coated with a hot melt adhesive, where the adhesive becomes tacky only at an elevated temperature (e.g. 80 °C). The deposition of ink to print the electrodes and other structures is quite easy with this system but it can present problems during the lamination step. The glue layer becomes substantially liquid at elevated temperatures with the consequence that the printed structure looses shape and gets stretched and deformed. Such deformation is not only a cosmetic problem for an electrode, it changes the electrode surface (which is directly proportional to the response signal) as well as the internal resistance of the material and the electro-catalytic properties.

Apart form the foregoing problems, there is the additional problem of glue entering and clogging or misshaping the channel. For the chip described above, the most advantageous process is an adhesive-free thermal bonding process of the pre-printed foil to the chip base plate. The bonding happens at an elevated temperature with a stamping tool or a hot roller press. The temperature is close to the glass transition temperature is to temperature (T_{g}) of the polymer thus the low molecular weight portion of the polymer will become mobile and tacky while the high molecular weight portion of the polymer still supports the integrity of the foil or film. The low molecular weight portion of the polymer will bond both pieces (base plate and foil with electrodes) together, additionally it will follow the shape of the printed electrodes, which can be between 5 and 30 µm thick. Therefore, one does not see a leakage between base plates and printed areas. Ideal bonding is achieved with the same thermoplastic polymers such as polystyrene on polystyrene or polycarbonate on polycarbonate. However, with the right regime and temperature/pressure combination polycarbonate can be bonded on polystyrene as well. But duro-plastic (non thermoplastic) materials are not suitable for such a process.

### 4. Channel Switching Mechanism

The switching mechanism 404 allows the replacement of a used electrochemical system after a predetermined period of time. Generally, an electrochemical system is prone to fouling due to precipitation or adsorption of proteins or other species on the electrode surface.

In the past, many different systems have been developed to overcome this problem. A classical example for such a system is the mercury-drop-electrode, where the mercury provides the electrode surface but every second the drop of mercury is replaced. Thus, the system never undergoes a continuous fouling process of a solid-state electrode. The renewal of the electrode surface is the most efficient but also the most dramatic strategy to avoid fouling. However, this same concept is used with all current disposable diagnostic glucose strips, where the test strip is disposed after a relatively short working time (5 to 15 seconds depending on the device).

A different strategy is the protection of the electrode by a semi permeable membrane, which allows the analyte to pass through to the electrode but not a large protein (or a red blood cell). This concept is adopted in classical oxygen electrodes.

The chip 400 uses a mixture of both concepts: a plurality of electrodes in different channels as well as an electrode protected by screen printable membrane (see German patent DE10052066.9 and related International patent application PCT/EP01/12073). This allows the sensor electrodes to operate for several hours before the fouling shows a significant effect on the results and before the controller is switching to the next channel.

Figure 21 shows a schematic diagram of the switching mechanism 404. At the beginning of a measurement, all channels 402 are filled with air and the entrance of fluid into the channel 402 from the distribution depression 416 is blocked due to the gas back pressure. The switching system 404 includes electrodes 422 separate from the electrochemical detector electrodes 418, 420. The switching electrodes 404 allow local heating of a very small area on the thin foil such as polystyrene 424 overlying a well 426. The well 426 is in fluid flow communication with the end of the reservoir 406 except that the fluid flow is normally blocked by the foil 424. Due to this blockage, air cannot escape from the channel 402 and fluid from the distribution depression 416 cannot flow into the channel 402. Upon heating of the foil, this blockage is removed thus allowing the fluid to enter the channel.

The temperature at the center point of the overlying electrodes 422 (i.e., overlying the foil 424 above the well 426) very quickly rises over the glass transition temperature (T_{g}) of the foil in response to an application of a small current in the range of some 1/1000 Ampere with the effect that the foil 424 forms an opening above the hydrophobic well 426 allowing air in the channel 402 and reservoir 406 to vent into the well 426. In this event, the gas pressure in the channel 402 is relieved and fluid can flow from the distribution depression 416 and into the channel 402.

The hydrophobicity of the well 426 prevents the sample fluid from flowing into the well 426 and leaking out of an opening formed in the film by reason of the energized electrodes 404. After the waste reservoir 406 is filled completely, the controller opens a subsequent channel 402 by energizing its switching electrodes 422 to permit air to escape from the channel 402 and for the subsequent channel 402 to fill with body fluid. It should be appreciated that other methods for switching between channels and controlling fluid flow could be used and the invention is not limited by the above method

### 5. Flow Rate Detection

To assure a safe operation, it is preferable the controller determine if the waste reservoir 406 is filled, is still filling at a constant rate, or if the needle 410 has dislodged from the cutaneous layer of the patient and the continuous sample stream has stopped. To achieve this, the waste reservoir 406 can be equipped with two electrodes (not shown) at the top and the bottom of the reservoir. Not in electrical contact with the sample fluid, these electrodes could measure the change in capacitance while the air inside the structure is slowly exchanged with sample fluid. The rate of the capacitance change will be directly proportional to the flow rate of the sample fluid and allow a close monitoring of the ongoing extraction.

### 6. Semi-Continuous Monitoring Alternative

An alteration of the foregoing is the scheduled measurement of discrete glucose values. In this case the waste reservoir is just large enough to replace the dead volume of the needle and sampling module but instead of for example twelve channels the chip contains a higher number of channels (e.g. 72 or 144 channels). These structures would support a 12-hour or 24-hour measurement cycle with a discrete glucose test very 10 minutes. An advantage of the semi-continuous method would be that it would not be necessary to use a cross-linked hydrogel due to the fact that migration of the reagents into the fluid sample over time would no longer an issue. Consequently, conventional enzyme inks such as disclosed in US5708247 could be used.

It will be appreciated by those skilled in the art that whilst some of the examples of the concepts disclosed herein have been described in greater detail, there are many different variations and modifications to these possible.

## Claims

1. A device (2) for measuring the concentration of an analyte in a bodily fluid, comprising skin penetration means (4), said penetration means (4) having a length sufficiently short so as to penetrate only the cutaneous layer of skin without penetrating the sub-cutaneous layer, such a length being less than 2 mm, and a support member (3a), said support member (3a) comprising an analyte sensing site and a microchannel (8) for conducting said body fluid from said penetration member (4) to said analyte sensing site, and wherein said penetration means (4) is integral with said support member (3a),
said device being arranged to take measurements at predetermined intervals,
said device further comprising flow control means (12) for influencing the flow of fluid to a sensing means (11),
**characterised in that** the flow control means (12) comprises a hydrophobic gate (612;128) disposed in a microchannel (8) that is configured to arrest the flow of fluid along the microchannel.

2. A device as claimed in claim 2 comprising a microneedle for extracting interstitial fluid.

3. A device as claimed in any of claims 1 or 2 which is adapted to measure glucose concentration.

4. A device as claimed in any preceding claim comprising analyte sensing means including a mediated amperometric enzyme electrode.

5. A device as claimed in claim 4 adapted to utilise ferrocene-mediated electron transfer from a glucoseoxidase catalysed reaction.

6. A device as claimed in any preceding claim comprising a plurality of analyte sensing means.

7. A device as claimed in any preceding claim which is arranged to measure an analyte concentration directly.

8. A device as claimed in any preceding claim which is suitable for attachment to the skin of a subject for measuring the concentration of an analyte in blood or interstitial fluid of the subject.

9. A device as claimed in claim 8 comprising means for attaching the device to the skin.

10. A device as claimed in any preceding claim comprising means for changing the hydrophobicity of said gate.

11. A device as claimed in claim 10 comprising a hydrophilic base material covered by a hydrophobic material and further comprising means for exposing said hydrophilic base material.

12. A device as claimed in any preceding claim comprising pumping means arranged to apply a pressure to liquid in said microchannel (8) sufficient to breach said hydrophobic gate.

13. A device as claimed in any preceding claim comprising a plurality of analyte sensing means (11), said device being arranged to direct fluid sequentially to each of the sensing means (11).

14. A device as claimed in claim 13 comprising or being adapted to interface with, control means for generating signals to control said direction of the fluid to the sensing means.

15. A device as claimed in claim 14 comprising said control configured so as to allow a user to specify when a measurement is to be made.

16. A device as claimed in claim 14 or 15 comprising colorimetric transduction means.

17. A device as claimed in any of claims 13 to 16 comprising a common fluid collection region arranged so as in use to be in fluid communication with a body fluid to be measured.

18. A device as claimed in claim 17 wherein each of said sensing means (11) is in selective fluid communication with said common collection region.

19. A device as claimed in any of claims 13 to 18 comprising a sensing means (11) for making a substantially continuous measurement of the concentration of a substance.

20. A device as claimed in any preceding claim which is suitable for attachment to a user's skin comprising transfer means for transferring said a body fluid from the user's body to a sensitive part of the device.

21. A device as claimed in claim 20 wherein said transfer means is arranged to transfer a fluid to the upstream end of a microchannel.

22. A device as claimed in claim 20 or 21 wherein said transfer means comprises a needle.

23. A device as claimed in claim 22 wherein said needle is a microneedle of such a length that it penetrates the cutaneous layer of skin, but not the sub-cutaneous layer.

24. A device as claimed in claim 22 or 23 wherein the needle comprises an aperture on a side surface thereof.

25. A device as claimed in any of claims 20 to 24 wherein said transfer means is invasive or semi-invasive, the device further comprising means (5) for applying pressure to the user's skin in the region of penetration by said transfer means.

26. A device as claimed in any preceding claim comprising display means for displaying the concentration of an analyte being measured.

27. A device as claimed in claim 26 wherein said display means is separate from the rest of the device and receives data therefrom by telemetry.

28. A device as claimed in any preceding claim comprising means for administering a substance to a user on the device on the basis of a measured analyte concentration.

29. A device as claimed in any preceding claim comprising electro-osmotic flow control means comprising a plurality of driving electrodes, said driving electrodes being provided substantially on one side of a channel.

30. A device as claimed in claim 29 wherein said driving electrodes extend circumferentially around an arcuate wall of the channel.

31. A device as claimed in any preceding claim comprising a main support member and a second substrate laminated to the main support member, said second substrate layer having one or more electrodes formed thereon.

32. A device as claimed in claim 31 wherein said second substrate is arranged to close a channel provided on the main support member.

33. Apparatus for measuring an analyte optically comprising a device as claimed in any preceding claim for measuring said analyte and a separate test meter including light sensitive means, wherein said measuring device and said test meter are arranged such that in use the light sensitive means views said analyte along a channel of the measuring device.

## Patentansprüche

1. Vorrichtung (2) zur Messung der Konzentration eines Analyten in einer Körperflüssigkeit, umfassend ein Hauteinstechmittel (4), wobei das Einstechmittel (4) eine ausreichend kurze Länge aufweist, um nur in die Haut einzustechen ohne die Unterhaut zu penetrieren, wobei diese Länge weniger als 2 mm beträgt, und ein Stützmittel (3a), wobei das Stützmittel (3a) eine Analytenmessstelle und einen Mikrokanal (8) zum Durchleiten der Körperflüssigkeit von dem Einstechmittel (4) zur Analytenmessstelle umfasst und worin das Einstechmittel (4) mit dem Stützelement (3a) einstückig ist,
worin die Vorrichtung zur Durchführung von Messungen in vorbestimmten Intervallen angeordnet ist,
worin die Vorrichtung ferner ein Durchflusskontrollmittel (12) zur Beeinflussung der Strömung der Flüssigkeit zu einem Messmittel (11) umfasst,
**dadurch gekennzeichnet, dass** das Durchflusskontrollmittel (12) eine hydrophobes Schranke (612; 128) umfasst, die in einem Mikrokanal (8) angeordnet ist und zum Anhalten der Flüssigkeitsströmung entlang des Mikrokanals ausgelegt ist.

2. Vorrichtung nach Anspruch 2, umfassend eine Mikronadel zur Extraktion von Interstitialflüssigkeit.

3. Vorrichtung nach einem der Ansprüche 1 oder 2, die zur Messung der Blutzuckerkonzentration ausgelegt ist.

4. Vorrichtung nach einem der vorhergehenden Ansprüche, umfassend ein Analytenmessmittel mit einer vermittelten amperometrischen Enzymelektrode.

5. Vorrichtung nach Anspruch 4, die zur Verwendung von Ferrocen-vermitteltem Elektronentransfer aus einer durch Glucoseoxidase katalysierten Reaktion ausgelegt ist.

6. Vorrichtung nach einem der vorhergehenden Ansprüche, umfassend eine Vielzahl von Analytenmessmitteln.

7. Vorrichtung nach einem der vorhergehenden Ansprüche, die zur direkten Messung einer Analytenkonzentration angeordnet ist.

8. Vorrichtung nach einem der vorhergehenden Ansprüche, die zur Befestigung an der Haut einer Person zur Messung der Konzentration eines Analyten im Blut oder in der Interstitialflüssigkeit der Person geeignet ist.

9. Vorrichtung nach Anspruch 8, umfassend ein Mittel zur Befestigung der Vorrichtung an der Haut.

10. Vorrichtung nach einem der vorhergehenden Ansprüche, umfassend ein Mittel zur Veränderung der Hydrophobie der Schranke.

11. Vorrichtung nach Anspruch 10, umfassend ein hydrophiles Basismaterial, das von einem hydrophoben Basismaterial bedeckt ist, und ferner umfassend ein Mittel zum Freilegen des hydrophilen Basismaterials.

12. Vorrichtung nach einem der vorhergehenden Ansprüche, umfassend ein Pumpmittel zum Aufbringen von ausreichendem Druck auf Flüssigkeit in dem Mikrokanal (8) zum Durchbrechen der hydrophoben Schranke.

13. Vorrichtung nach einem der vorhergehenden Ansprüche, umfassend eine Vielzahl von Analytenmessmitteln (11), worin die Vorrichtung zum sequentiellen Leiten der Flüssigkeit zu jedem der Messmittel (11) angeordnet ist.

14. Vorrichtung nach Anspruch 13, umfassend ein oder ausgelegt zur Verbindung mit einem Kontrollmittel zur Erzeugung von Signalen zur Kontrolle der Leitung der Flüssigkeit zu dem Messmittel.

15. Vorrichtung nach Anspruch 14, umfassend die Kontrolle, die so konfiguriert ist, das ein Anwender vorgeben kann, wann eine Messung erfolgen soll.

16. Vorrichtung nach Anspruch 14 oder 15, umfassend ein kolorimetrisches Übertragungsmittel.

17. Vorrichtung nach einem der Ansprüche 13 bis 16, umfassend eine gemeinsame Flüssigkeitssammelregion, die im Gebrauch mit einer zu messenden Körperflüssigkeit in Flüssigkeitsverbindung steht.

18. Vorrichtung nach Anspruch 17, worin jedes Messmittel (11) mit der gemeinsamen Sammelregion in selektiver Flüssigkeitsverbindung steht.

19. Vorrichtung nach einem der Ansprüche 13 bis 18, umfassend ein Messmittel (11) zur Durchführung einer im Wesentlichen kontinuierlichen Messung der Konzentration einer Substanz.

20. Vorrichtung nach einem der vorhergehenden Ansprüche, die zur Befestigung an der Haut eines Anwenders geeignet ist und ein Transfermittel zur Weiterleitung der Körperflüssigkeit von Körper des Anwenders zu einem empfindlichen Teil der Vorrichtung umfasst.

21. Vorrichtung nach Anspruch 20, worin das Transfermittel zur Weiterleitung einer Flüssigkeit zum stromaufwärts gelegenen Ende eines Mikrokanals angeordnet ist.

22. Vorrichtung nach Anspruch 20 oder 21, worin das Transfermittel eine Nadel umfasst.

23. Vorrichtung nach Anspruch 22, worin die Nadel eine Mikronadel ist, die eine solche Länge aufweist, dass sie in die Haut, aber nicht in die Unterhaut eindringt.

24. Vorrichtung nach Anspruch 22 oder 23, worin die Nadel eine Öffnung auf einer Seitenfläche umfasst.

25. Vorrichtung nach einem der Ansprüche 20 bis 24, worin das Transfermittel invasiv oder nicht-invasiv ist, worin die Vorrichtung ferner ein Mittel (5) zum Aufbringen von Druck auf die Haut des Anwenders in der Eindringregion des Transfermittels umfasst.

26. Vorrichtung nach einem der vorhergehenden Ansprüche, umfassend ein Anzeigemittel zum Anzeigen der Konzentration eines gemessenen Analyten.

27. Vorrichtung nach Anspruch 26, worin das Anzeigemittel vom Rest der Vorrichtung getrennt ist und durch Telemetrie Daten von ihr erhält.

28. Vorrichtung nach einem der vorhergehenden Ansprüche, umfassend ein Mittel zur Verabreichung einer Substanz an einen Anwender an der Vorrichtung auf Basis einer gemessenen Analytenkonzentration.

29. Vorrichtung nach einem der vorhergehenden Ansprüche, umfassend ein elektroosmotisches Durchflusskontrollmittel, umfassend eine Vielzahl von Antriebselektroden, worin die Antriebselektroden im Wesentlichen auf einer Seite eines Kanals vorgesehen sind.

30. Vorrichtung nach Anspruch 29, worin sich die Antriebselektroden um den Umfang einer bogenförmigen Wand des Kanals erstrecken.

31. Vorrichtung nach einem der vorhergehenden Ansprüche, umfassend ein Hauptstützelement und ein zweites Substrat, das auf das Hauptstützelement laminiert ist, worin die zweite Substratschicht ein oder mehr darauf geformte Elektroden aufweist.

32. Vorrichtung nach Anspruch 31, worin das zweite Substrat zum Schließen eines Kanals auf dem Hauptstützelement angeordnet ist.

33. Gerät zur optischen Messung eines Analyten, das eine Vorrichtung nach einem der vorhergehenden Ansprüche zur Messung des Analyten und ein separates Testmessgerät mit einem lichtempfindlichen Mittel umfasst, worin die Messvorrichtung und das Testmessgerät so angeordnet sind, dass im Gebrauch das lichtempfindliche Mittel den Analyten entlang eines Kanals der Messvorrichtung betrachtet.

## Revendications

1. Dispositif (2) pour mesurer la concentration d'un analyte dans un fluide corporel, comprenant un moyen de pénétration de la peau (4), ledit moyen de pénétration (4) ayant une longueur suffisamment courte pour pénétrer uniquement la couche cutanée de la peau sans pénétrer la couche sous-cutanée, une telle longueur étant inférieure à 2 mm, et un élément formant membre support (3a), ledit élément formant membre support (3a) comprenant un site de détection d'analyte et un microcanal (8) pour conduire ledit fluide corporel depuis ledit élément formant membre de pénétration (4) jusqu'audit site de détection d'analyte, et où ledit moyen de pénétration (4) est intégré audit élément formant membre support (3a),
ledit dispositif étant conçu pour prendre des mesures à des intervalles prédéterminés,
ledit dispositif comprenant en outre un moyen de contrôle d'écoulement (12) pour influencer l'écoulement du fluide vers un moyen de détection (11),
**caractérisé en ce que** le moyen de contrôle d'écoulement (12) comprend une porte hydrophobe (612 ; 128) disposée dans un microcanal (8) qui est configurée pour arrêter l'écoulement du fluide le long du microcanal.

2. Dispositif tel que revendiqué dans la revendication 1, comprenant une micro-aiguille pour extraire du fluide interstitiel.

3. Dispositif tel que revendiqué dans l'une quelconque des revendications 1 ou 2, qui est conçu pour mesurer une concentration de glucose.

4. Dispositif tel que revendiqué dans l'une quelconque des revendications précédentes, comprenant un moyen de détection d'analyte comprenant une électrode enzymatique ampérométrique assistée.

5. Dispositif tel que revendiqué dans la revendication 4, conçu pour utiliser un transfert électronique assisté par ferrocène à partir d'une réaction catalysée de glucose oxydase.

6. Dispositif tel que revendiqué dans l'une quelconque des revendications précédentes, comprenant une pluralité de moyens de détection d'analyte.

7. Dispositif tel que revendiqué dans l'une quelconque des revendications précédentes, qui est conçu pour mesurer une concentration d'analyte directement.

8. Dispositif tel que revendiqué dans l'une quelconque des revendications précédentes, qui est approprié pour être fixé à la peau d'un sujet pour mesurer la concentration d'un analyte dans le sang ou dans un fluide interstitiel du sujet.

9. Dispositif tel que revendiqué dans la revendication 8 comprenant un moyen pour fixer le dispositif à la peau.

10. Dispositif tel que revendiqué dans l'une quelconque des revendications précédentes, comprenant un moyen pour changer l'hydrophobicité de ladite porte.

11. Dispositif tel que revendiqué dans la revendication 10, comprenant un matériau de base hydrophile recouvert d'un matériau hydrophobe et comprenant en outre un moyen pour exposer ledit matériau de base hydrophile.

12. Dispositif tel que revendiqué dans l'une quelconque des revendications précédentes, comprenant un moyen de pompage conçu pour appliquer une pression à un liquide dans ledit microcanal (8) suffisante pour rompre ladite porte hydrophobe.

13. Dispositif tel que revendiqué dans l'une quelconque des revendications précédentes, comprenant une pluralité de moyens de détection d'analyte (11), ledit dispositif étant conçu pour diriger un fluide en séquence vers chacun des moyens de détection (11).

14. Dispositif tel que revendiqué dans la revendication 13, comprenant ou étant conçu pour être interconnecté avec un moyen de contrôle pour générer des signaux pour contrôler ladite direction du fluide vers les moyens de détection.

15. Dispositif tel que revendiqué dans la revendication 14, comprenant ledit contrôle qui est configuré de manière à permettre à un utilisateur de spécifier quand une mesure doit être effectuée.

16. Dispositif tel que revendiqué dans la revendication 14 ou la revendication 15, comprenant un moyen de transduction colorimétrique.

17. Dispositif tel que revendiqué dans l'une quelconque des revendications 13 à 16, comprenant une région de collecte de fluide commune conçue de manière à être, en utilisation, en communication fluide avec un fluide corporel à mesurer.

18. Dispositif tel que revendiqué dans la revendication 17, dans lequel chacun desdits moyens de détection (11) est en communication fluide sélective avec ladite région de collecte commune.

19. Dispositif tel que revendiqué dans l'une quelconque des revendications 13 à 18, comprenant un moyen de détection (11) destiné à effectuer une mesure sensiblement continue de la concentration d'une substance.

20. Dispositif tel que revendiqué dans l'une quelconque des revendications précédentes, qui est approprié pour être fixé sur la peau d'un utilisateur et comprenant un moyen de transfert pour transférer ledit fluide corporel du corps de l'utilisateur vers une partie sensible du dispositif.

21. Dispositif tel que revendiqué dans la revendication 20, dans lequel ledit moyen de transfert est conçu pour transférer un fluide vers l'extrémité amont d'un microcanal.

22. Dispositif tel que revendiqué dans la revendication 20 ou la revendication 21, dans lequel ledit moyen de transfert comprend une aiguille.

23. Dispositif tel que revendiqué dans la revendication 22, dans lequel ladite aiguille est une micro-aiguille d'une longueur telle qu'elle pénètre la couche cutanée de la peau mais pas la couche sous-cutanée.

24. Dispositif tel que revendiqué dans la revendication 22 ou la revendication 23, dans lequel l'aiguille comprend une ouverture sur sa surface latérale.

25. Dispositif tel que revendiqué dans l'une quelconque des revendications 20 à 24, dans lequel ledit moyen de transfert est invasif ou semi-invasif, le dispositif comprenant en outre un moyen (5) pour appliquer une pression au niveau de la peau de l'utilisateur dans la région de pénétration par ledit moyen de transfert.

26. Dispositif tel que revendiqué dans l'une quelconque des revendications précédentes, comprenant un moyen d'affichage pour afficher la concentration d'un analyte étant mesuré.

27. Dispositif tel que revendiqué dans la revendication 26, dans lequel ledit moyen d'affichage est séparé du reste du dispositif et reçoit des données de celui-ci par télémétrie.

28. Dispositif tel que revendiqué dans l'une quelconque des revendications précédentes, comprenant un moyen pour administrer une substance à un utilisateur du dispositif sur la base de la concentration d'un analyte mesuré.

29. Dispositif tel que revendiqué dans l'une quelconque des revendications précédentes, comprenant un moyen de contrôle d'écoulement électro-osmotique comprenant une pluralité d'électrodes de commande, lesdites électrodes de commande étant pourvues sensiblement d'un côté d'un canal.

30. Dispositif tel que revendiqué dans la revendication 29, dans lequel lesdites électrodes de commande s'étendent de manière circonférentielle autour d'une paroi courbe du canal.

31. Dispositif tel que revendiqué dans l'une quelconque des revendications précédentes, comprenant un élément formant membre support principal et un second substrat laminé sur l'élément formant membre support principal, ladite couche du second substrat comportant une ou plusieurs électrodes formées sur celle-ci.

32. Dispositif tel que revendiqué dans la revendication 31, dans lequel ledit second substrat est conçu pour fermer un canal pourvu sur l'élément formant membre support principal.

33. Appareil pour mesurer un analyte comprenant optiquement un dispositif tel que revendiqué dans l'une quelconque des revendications précédentes destiné à mesurer ledit analyte et un testeur séparé comprenant un moyen photosensible, où ledit dispositif de mesure et ledit testeur sont conçus de manière à ce qu'en utilisation le moyen photosensible voit ledit analyte le long d'un canal du dispositif de mesure.
